Europäisches Patentamt

(19) European Patent Office

Office européen des brevets

(11) Publication number: **0 151 519**
**B1**

(12) **EUROPEAN PATENT SPECIFICATION**

(45) Date of publication of patent specification: 29.03.89

(21) Application number: 85300321.8

(22) Date of filing: 17.01.85

(51) Int. Cl.⁴: **F 16 L 37/00,** F 16 L 47/00, A 61 M 5/00

(54) **Quick disconnect tube coupling.**

(30) Priority: 25.01.84 US 573545

(43) Date of publication of application:
14.08.85 Bulletin 85/33

(45) Publication of the grant of the patent:
29.03.89 Bulletin 89/13

(84) Designated Contracting States:
AT BE CH DE FR GB IT LI LU NL SE

(56) References cited:
EP-A-0 057 057
GB-A-1 193 759
US-A-4 140 654

(73) Proprietor: E.R. Squibb & Sons, Inc.
Lawrenceville-Princeton Road
Princeton, N.J. 08540 (US)

(72) Inventor: Campbell, Randolph E.
223 Maple Avenue
Red Bank New Jersey (US)
Inventor: Spier, I. Martin
50 Park Avenue
New York (US)

(74) Representative: Cook, Anthony John et al
D. YOUNG & CO. 10, Staple Inn
London, WC1V 7RD (GB)

Courier Press, Leamington Spa, England.

**Description**

This invention relates to an improved tube coupling for connecting to two tube lengths which coupling provides an effective liquid seal when the tube lengths are connected. More particularly, the invention relates to tube couplings for medical use although it is not limited to medical applications.

In the prior art many tube couplings are shown comprising a male body and a female body and which provide for a positive locking capability when joining the male and female bodies together.

To provide a liquid seal various approaches are used in the prior art, but one of the most common comprises placement of separately manufactured O-rings in annular grooves surrounding a portion of the male body which portion is inserted in a corresponding cavity of the female body. One example of such a construction is seen in GLADIEUX U.S. Patent (US—A—4 240 654). O-rings generally function by having a larger outer diameter when placed in the grooves on the male member than the outer diameter of the corresponding portion of the male member. The O-rings are made of a compressible material such as rubber and when inserted in the female cavity, they are compressed against the relatively smooth interior surface thereof to form a liquid seal.

The various prior art devices require separate approaches for providing a locking mechanism and a liquid seal. For example, Applicant has used a pair of latching arms hold the two coupling parts together, and a different arrangement embodying latching arms is shown in British Patent 1 193 759. Further, the prior art devices require some form of separate action to remove the locking action of the locking device; and require the inclusion of separately manufactured O-rings in the assembly of the tube coupling.

An aim of the present invention is to provide an improved tube coupling by means of an improved method of making same.

The present invention comprises a method of making a coupling as defined in claim 1. In a preferred embodiment, the at least one peripheral member is moulded within a groove in a female body surrounding a hollow plug end portion of the male body. Where the male body is further made of a synthetic plastic material formed in a mold it further comprises an alignment means for properly aligning the male body after forming thereof to accept forming of the peripheral member in the groove by subsequent insert molding.

The tube coupling preferably comprises both a locking and a liquid sealing means. The locking and sealing means comprises at least a pair of spaced apart peripheral members which are disposed within groves in one of the bodies, a first one of the peripheral members disposed to be compressed against the surface of the remaining body to form a liquid seal therewith. The second one of the peripheral members fits within a groove located within the mating body when the male and female bodies are matingly engaged.

In one embodiment of the coupling, a pair of spaced apart peripheral members are disposed within grooves surroundng a hollow plug end portion of the male body. One of the peripheral members is disposed to be compressed against the interior surface of a receiving end portion of the female body to provide a liquid seal while the other peripheral member is disposed to fit within a groove located within the interior surface of the receiving end portion of the female body. Alternatively, the peripheral members could be disposed within the interior surface of the receiving end portion and an annular groove for receiving one of the peripheral members surrounds the plug end portion of the male body.

In the preferred embodiment of the coupling, the peripheral member which provides the primary liquid seal with the interior surface of the mating body has a substantially rectangular cross section, while the peripheral member providing a substantial locking action has a part-circular cross section.

In one embodiment, the receiving end portion of the female body is tapered.

Preferably, the locking and sealing means are made of an elastomeric thermoplastic resin material and is integrally formed with either the male or female body made from a high density polyethylene or polypropylene.

Other features and advantages of the invention will become apparent from the following description read in conjunction with the drawings in which:

Figure 1 is a longitudinal view of the male and female bodies of the present invention, the female body being shown in cross section and the male body being shown partially in cross section.

Figure 2 is an enlarged cross-sectional view of a portion of the male body of Figure 1 taken along the lines and arrows 2.

Figure 3 is a longitudinal view of an an alternative embodiment showing a portion of the male and female bodies in a view similar to that of Figure 1.

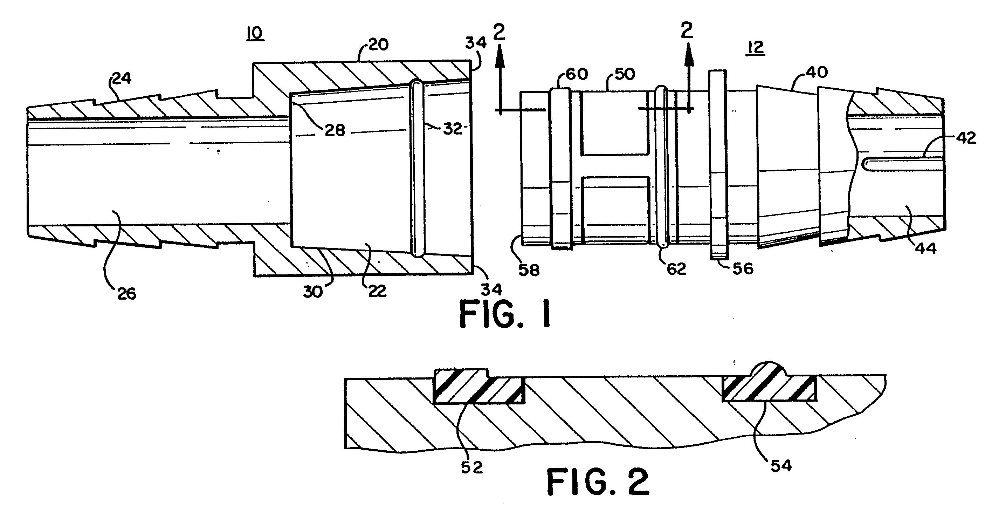

Referring now to Figure 1 the male and female bodies designated generally 12 and 10 respectively are shown separated but located along a common longitudinal axis. Female body 10 is shown in cross section and comprises a tubular shaped receiving end portion 20 which defines an interior region 22. Opposite from receiving end portion 20 and integrally connected therewith is a tube connecting portion 24 with a tapered saw tooth shaped outer surface. The tube connecting portion 24 is adapted to be inserted into a length of tube and the tapered saw tooth outer surface provides a gradually increasing friction fit between the female body and the length of tube while preventing excessive distortion and deformation of the tubing. The entry portion of the length of tube is in fairly continuous contact with the saw tooth outer surface. The hollow interior 26 of the tube connecting portion 24 communicates with both the interior of the length of tube and the interior region 22.

In Figure 1, the interior surface of the receiving end portion 20 is tapered, that is, the cross-sectional diameter of the interior region 22 at the open end of the receiving end portion 20 is larger than the parallel and spaced apart cross-sectional diameter of the interior region 22 defined at the rim 28 of the receiving end portion 20, and the wall 30 of the interior surface slopes linearly from the cross-sectional diameter at the open end to inner cross-sectional diameter at the rim 28. The interior surface of the receiving end portion 20 further comprises an annular groove 32.

Male body 12 comprises a tube connecting portion 40 similar in shape and purpose to the tube connecting portion 24 of the female body 10. However, it further comprises a pair of ledges only one of which 42 is shown which ledges project inwardly into the hollow interior 44 of the tube connecting portion 40. The ledges 42 are in the preferred embodiment located on a common diameter of the tube connecting portion 40.

Male body 12 further comprises a tubular shaped plug end portion 50 integrally formed with the tube connecting portion 40 and adapted to be inserted into the interior region 22 of the receiving end portion 20. The interior of the plug end portion 50 communicates with the hollow interior 44 of the tube connecting portion 40 which in turn communicates with the interior of a length of tube when the tube connecting body 40 is inserted therein.

It should be appreciated that the term "tubular shaped" as used to describe the receiving end portion and the plug end portion includes hollow cylinders or bodies of various shaped cross sections including circular, rectangular and square cross sections.

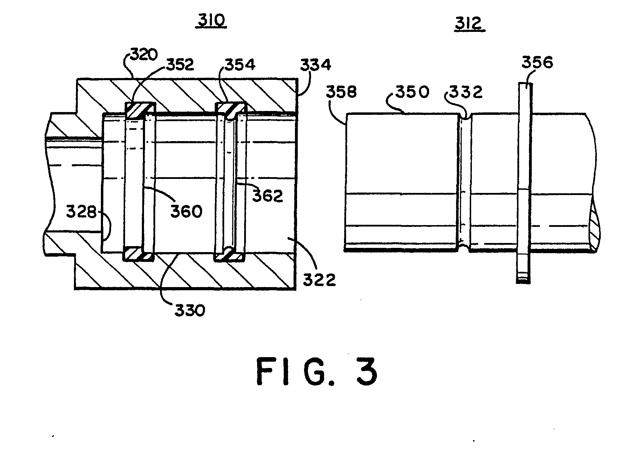

The outer diameter (OD) of the generally cylindrically shaped plug end portion is uniform throughout its length except at grooves 52 and 54, shown in Figure 3, and except for the annular ridge 56. The outer diameter of the plug end portion 50 is slightly less than the cross-sectional diameter of the interior region 22 at rim 28. For example, the outer diameter of the plug end portion in the preferred embodiment is approximately ·912 cm (0·359") while the cross-sectional diameter of the interior region 22 at the open end of the receiving end portion is approximately 1·011 cm (0·398") and the cross-sectional diameter at rim 28 approximately ·940 cm (0·370").

The longitudinal length of the plug end portion from the open end 58 to the annular ridge 56 is substantially equal to the longitudinal length of the interior region 22 from the inner cross-sectional diameter at rim 28 to the outer edge of the receiving end portion 20. Annular ridge 56 contacts the outer surface 34 of the receiving end portion 20 as the open end 58 of the plug end portion 50 contacts the rim 28.

Figure 2 is an enlarged cross section of a portion of the male body in Figure 1 taken along the lines and arrows 2 in Figure 1. The plug end portion 50 includes the annular rectangularly shaped grooves 52 and 54. In the preferred embodiment, the grooves are filled by inset molding with an elastomeric thermoplastic resin material which forms a pair of peripheral members 60 and 62. Ledges 42 assist in aligning the male body during the insert molding step. Inspection of Figures 1 and 2 show that the OD of both peripheral members 60 and 62 exceeds the OD of the plug end portion 50, with the peripheral member 62 having a slightly larger OD than the peripheral member 60. In the preferred embodiment, the OD of the peripheral member 60 is ·973 cm (0·383") and the OD of the peripheral member 62 is ·993 cm (0·391").

While the preferred material for the peripheral members 60 and 62 is an elastomeric thermoplastic resin, the preferred material for the male body is any rigid plastic material suitable for the intended application of the coupling. In medical applications a suitable rigid plastic material comprises a high density polyethylene or polypropylene.

An alternative method for forming the peripheral members 60 and 62 is as follows: the male body is formed by injection molding and, before the plastic material of which the male body is made sets, the peripheral members are formed by injection molding of an elastomeric thermoplastic resin. The plastic resin melt fills the male body mold in the groovs 52 and 54 and mixes with the plastic material of which the male body is composed at the mold interface surrounding the grooves 52 and 54. Here the primary melt of the male body intimately coalesces with the secondary melt of the thermoplastic resin to form a chemical and mechanical bond between the two materials. The injection molding step for both the male body and the peripheral members may take place simultaneously where an appropriate mold for the male body including the peripheral members is fabricated and both primary and secondary mold injection stations are established. Alternatively, the injection molding step of the male body occurs first followed soon after by the injection molding step for the peripheral members, the latter injection molding step occuring before the melt composition of the male body has set.

Referring once again to Figure 1, as the male body is inserted into the female body, the peripheral member 60 coms in contact with the wall 30 and is gradually compressed against the wall 30 as the male body is fully inserted. Deformation of the peripheral member 60 against the wall 30 forms a liquid seal. As the front edge 58 of the male body approaches the rim 28, the peripheral member 62 begins to seat itself within annular groove 32. The maximum dimension of groove 32 is approximately equal to the OD of the peripheral member 62. The curved shape of the peripheral member 62 aids in seating member 62 within annular groove 32. Positioning of member 62 in groove 32 acts to lock the female and male bodies together. Also, member 62 seated in groove 32 acts as a further liquid seal. The peripheral member 62 in combination with a tight fitting groove 32 acts as a liquid sealing and locking means.

Hence, bodies 10 and 12 are quickly connected together in one pushing action applied along their common longitudinal axis. All that is required to disconnect the bodies is a pulling force of each body away from the other again along their common longitudinal axis. A sufficient force must be applied to overcome the seating of peripheral member 62 in groove 32. Since member 62 is compressible, it will eventually give way and leave groove 32 as the pulling force increases. However, the locking force is sufficient that under normal use the connector will not separate. Since the peripheral members 60 and 62 are preferably an elastomeric material they return to their original shape after the bodies 10 and 12 are disconnected.

The tapered interior surface assists in bringing the male and female bodies into mating engagement. The frictional force encountered with deformation of member 60 as it slides along wall 30 also aids in locking the bodies together. Also, the tapered interior aids in seating peripheral member 62 into groove 32.

With the tube coupling of the present invention, there is no latch or locking mechanism requiring a separate action to disconnect the male and female bodies such as a squeeze or twisting action. The peripheral members which provide a seal also act to create a locking action. This is possible because the peripheral members 60 and 62 are a compressible material which is formed integral with the rigid material of the male member. In the case where the peripheral members are formed by injection molding before the male body melt composition has set, the chemical and mechanical bond formed thereby acts to avoid separation of the peripheral members 60 and 62 from the grooves 52 and 54 respectively.

Figure 3 is an alternative embodiment 310 and 312 of a portion of the male and female bodies 10 and 12, respectively, of Figure 1. The bodies 310 and 312 are equipped with tube connecting portions identical to tube connecting portions 24 and 40, respectively, and they accordingly are not shown in Figure 3. Female body 310 comprises a receiving end portion 320 and a male body 312 comprises a plug end portion 350. However, instead of forming peripheral members in grooves on the external surface of the plug end portion 350, peripheral members 360 and 362 are formed within grooves in the interior surface of wall 330 of receiving end portion 320 by insert molding or simultaneous injection molding as described above. The cross-sectional diameter of the interior region 322 of the receiving end portion 320 is generally constant throughout its length from the interior rim 28 to the open end of the receiving end portion.

Spigot end portion 350 further comprises an annular groove 32 and an annular ridge 356. The cross-sectional diameter of the plug end portion 350 is generally constant throughout its length except for the annular groove 332. Also, the cross-sectional diameter of plug end portion 350 is generally less than then the cross-sectional area of the interior region 322. However, the inner diameter of both the peripheral members 360 and 362 are smaller than both the cross-sectional diameters of the interior region 322 and the plug end portion 350. As plug end portion 350 is inserted into the receiving end portion 320 the exterior surface of the plug end portion 350 frictionally contacts the peripheral members 360 and 362. The peripheral members 360 and 362 are made of a compressible material such as an elastomeric thermoplastic resin and as the plug end portion is inserted further into the interior region 322 they make a liquid seal with the exterior surface of the plug end portion 350. As the front end 358 of the plug end portion contacts the annular rim 328 the peripheral member 362 seats itself within the annular groove 332 of the plug end portion 350. At the same time annular ridge 356 engages the outer edge 334 of the receiving end portion 320.

The peripheral member 360 in the preferred embodiment has a rectangular cross section while the peripheral member 362 has a circular cross section. The circular cross section of peripheral member 362 aids in seating the peripheral member 362 and the annular groove 332.

The peripheral members 360 and 362 interacting with the plug end portion 350 act to create a liquid seal and a positive locking action without interferring with a quick disconnect capability of the tube coupler.

Claims

1. A method of making a coupling for connecting to two tube lengths to be coupled, the method comprising:
    providing a male body (12);
    providing a female body (10) for receiving at least a portion (50) of said male body (12) for a mating engagement therewith; and
    providing at least one peripheral member (62) of a relatively compressible material located within a groove (54) of one (12) of said bodies and configured and disposed to become compressed against a surface of the remaining one of said bodies when said male and female bodies are matingly coupled;
    the method being characterised in that the peripheral member (62) is molded within said groove (54) from a plastics material.

2. A method according to claim 1 wherein said one body comprises a synthetic plastics material formed in a mold and further comprises alignment means (42) for properly aligning said one body after forming to accept the subsequent insert molding of said at least one peripheral member in said groove.

3. A method according to claim 1 or 2 characterised in that at leat a pair of spaced apart peripheral members (60, 62) are molded within respective spaced grooves (52, 54) in one of said bodies.

4. A method according to claim 3 wherein said at least one pair of spaced apart peripheral members are formed within said gooves by insert molding.

5. A method according to claim 3 wherein said one body and said at least a pair of peripheral members are formed by injection molding carried out in such a way that injectoin molding of said peripheral members occurs after the injection molding of the said one body but before said one body has set.

6. A method according to any one of claims 1—5 in which said one body comprises a high density polyethylene and said at least a pair of peripheral members comprises an elastomeric thermoplastic resin.

## Patentansprüche

1. Verfahren zum Herstellen einer Kupplung zum Verbinden zweier zu verbindender Rohrstücke, wobei die Kupplung folgendes aufweist: einen Steckkörper (12),

einen Aufnahmekörper (10) zur Aufnahme wenigstens eines Abschnittes (50) des Steckkörpers (12) in Paßeingriff mit demselben, und

wenigstens ein Umfangsteil (62) aus einem relativ kompressiblen Material, das sich in einer Nut (54) einer (12) der Körper befindet, und das derart gestaltet und angeordnet ist, daß es gegen eine Fläche des anderen Körpers drückbar ist, wenn die Steck- und Aufnahmekörper passend verbunden sind,

dadurch gekennzeichnet, daß das Umfangsteil (62) in der Nut (54) aus einem Kunststoffmaterial gegossen wird.

2. Verfahren nach Anspruch 1, bei dem ein Körper ein synthetisches Kunststoffmaterial aufweist, das in einer Form geformt wird, und ferner Ausrichteinrichtungen (42) zum geeigneten Ausrichten dieses einen Körpers nach der Formung aufweist, um das anschließende Einsatzgießen des wenigstens einen Umfangsteils in der Nut aufzunehmen.

3. Verfahren nach Anspruch 1 oder 2, dadurch gekennzeichnet, daß wenigstens zwei im Abstand angeordnete Umfangsteile (60, 62) in zugeordnete und im Abstand liegende Nuten (52, 54) in einer der Körper gegossen werden.

4. Verfahren nach Anspruch 3, bei dem wenigstens zwei im Abstand angeordnete Umfangsteile in den Nuten durch Einsatzgießen ausgeformt werden.

5. Verfahren nach Anspruch 3, bei dem ein Körper und wenigstens zwei Umfangsteile durch Spritzgießen gebildet werden, das derart ausgeführt wird, daß das Spritzgiessen der Umfangsteile vorgenommen wird, nachdem das Spritzgießen des einen Körpers erfolgt ist, aber bevor sich dieser eine Körper verfestigt hat.

6. Verfahren nach einem der Ansprüche 1 bis 5, bei dem ein Körper ein hoch dichtes Polyethylen und die wenigstens zwei Umfangsteile ein elastomeres, thermoplastiches Harz aufweisen.

## Revendications

1. Procédé de fabrication d'un raccord destiné à être raccordé à deux tronçons de tube à réunir, ce raccord comprenant:

un corps mâle (12);

un corps femelle (10) destiné à recevoir au moins une partie (50) dudit corps mâle (12) en vue d'une prise mutuelle complémentaire; et

au moins un élément périphérique (62), en matière relativement compressible, placé dans une gorge (54) de l'un (12) desdits corps, et dont la configuration et la disposition sont telles qu'il sera comprimé contre une surface de l'autre desdits corps quand lesdits corps mâle et femelle sont en prise complémentaire;

caractérisé en ce que l'élément périphérique (62) est fait d'une matière plastique moulée à l'intérieur de ladite gorge (54).

2. Procédé selon la revendication 1, dans lequel ledit un corps comprend une matière plastique synthétique mise en forme dans un moule et comprend en outre un moyen d'alignement (42) permettant d'aligner correctement ledit un corps après mise en forme pour permettre le moulage rapporté ultérieur dudit au moins un élément périphérique dans ladite gorge.

3. Procédé selon la revendication 1 ou 2, caractérisé en ce qu'au moins deux éléments périphériques espacés (60, 62) sont moulés dans des gorges espacées respectives (52, 54) de l'un desdits corps.

4. Procédé selon la revendication 3, dans lequel lesdits au moins deux éléments périphériques espacés sont formés dans lesdites gorge par moulage rapporté.

5. Procédé selon la revendication 3, dans lequel ledit un corps et lesdits au moins deux éléments périphériques sont formés par moulage par injection, exécuté de telle sorte que le moulage par injection desdits éléments périphériques a lieu après le moulage par injection dudit un corps mais avant que ledit un corps ait durci.

6. Procédé selon l'une quelconque des revendications 1 à 5, dans lequel ledit un corps comprend un polyéthylène à haute densité et lesdits au moins deux éléments périphériques comprennent une résine thermoplastique élastomère.

FIG. 1

FIG. 2

FIG. 3